# EUROPEAN PATENT APPLICATION

(11) **EP 4 714 437 A1**
(43) Date of publication of application: **25.03.2026**
(21) Application number: 24938419.9
(22) Date of filing: 17.07.2024
(51) Int. Cl.: A61K 9/70, A61K 47/36, A61K 47/38, A61K 47/26

(54) **MICRO-BUBBLE FILM FOR REPLACEMENT OF TITANIUM DIOXIDE AND PREPARATION METHOD THEREFOR**

(30) Priority: 13.05.2024 CN 202410588528
(71) Applicant: Shanghai Advanced Pharmaceutical Engineering Research Center Co., Ltd., Shanghai 20120 (CN)
(72) Inventor: CHEN, Fang, Shanghai 201203 (CN); YANG, Liuliu, Shanghai 201203 (CN); LI, Mingyan, Shanghai 201203 (CN); ZHANG, Junqi, Shanghai 201203 (CN); WANG, Bing, Shanghai 201203 (CN)
(74) Representative: Bryn Aarflot AS
(86) International application number: PCT/CN2024/106001
(87) International publication number: WO 2025/236402

(57) **Abstract**

A micro-bubble film relating to the replacement of titanium dioxide and a preparation method therefor. The micro-bubble film is prepared mainly from the following ingredients: 0.01%-40% by mass of an active ingredient, 20%-80% by mass of a polymer film-forming material, 0.1%-20% by mass of a surfactant, and 2.5%-30% by mass of a thickening agent, wherein the polymer film-forming material comprises the pullulan polysaccharide as a main film-forming material and comprises an auxiliary film-forming material, and the mass ratio of the main film-forming material, the pullulan polysaccharide, to the auxiliary filmforming material is 1:3-3:1. Due to the physical effect of micro-bubbles scattering light beams, the film appears white and opaque. The white and opaque appearance, coupled with the particular ratio of the main film-forming material, the pullulan polysaccharide, to the auxiliary film-forming material, enables the prepared micro-bubble film to provide a new idea and a new solution to the replacement of titanium dioxide.

## Description

### RELATED APPLICATION

This application claims priority to the Chinese patent application filed on May 13, 2024, with the application number 2024105885289, entitled "MICRO-BUBBLE FILM FOR REPLACEMENT OF TITANIUM DIOXIDE AND PREPARATION METHOD THEREFOR", which is incorporated herein by reference in its entirety.

### TECHNICAL FIELD

The present disclosure relates to the technical field of film agents, specifically to a microbubble film agent as a substitute for titanium dioxide and a method for preparing the same.

### BACKGROUND

Film agents have garnered concern and attention in recent years due to their characteristics such as easyadministration, simple process, and good compliance. Typically, film agents require opaque and coloring treatments to provide recognizability and a good appearance, and to shield light, thereby protecting light-unstable components in the agent and improving drug quality. The most commonly used opaque coloring agent is titanium dioxide. Titanium dioxide is a white pigment with excellent whiteness, opacity, tinting strength, and extremely high chemical stability, and has been widely used for a long time in the fields of food, pharmaceuticals, health products, or cosmetics. It can achieve ideal coloring effects even at relatively low levels. Titanium dioxide can be used alone or in combination with other pigments or colorants such as ferric oxide to obtain vivid colors, further enhancing recognizability and aesthetics.

However, the safety of titanium dioxide is being questioned. In 2016, the French competent authority submitted a proposal to the European Chemicals Agency (ECHA) to classify titanium dioxide as a carcinogen. Subsequently, the Committee for Risk Assessment (RAC) passed a resolution the following year to classify titanium dioxide as a Category 2 carcinogen, including the hazard statement "H351 (inhalation)". On February 18, 2020, the European Commission, based on the RAC resolution, adopted Regulation (EU) 2020/217, providing a harmonized classification and labeling for titanium dioxide. According to this regulation, it is confirmed that titanium dioxide in powder form containing 1% or more of particles with a diameter equal to or less than 10 µm is carcinogenic to humans upon inhalation. On November 8, 2021, member states approved the European Commission's proposal to ban the use of titanium dioxide (E171) as a food additive from 2022. On January 14, 2022, the European Commission formally adopted the ban on the use of titanium dioxide as a food additive, stipulating a 6-month transition period. This ban officially came into full effect on August 7, 2022. Therefore, researchers are actively seeking alternative substances to titanium dioxide in the preparation of film agents.

In view of this, the present disclosure is specifically proposed.

### SUMMARY

A first object of the present disclosure is to provide a microbubble film agent as a substitute for titanium dioxide. This microbubble film agent utilizes the physical scattering effect of microbubbles on light to render the film white and opaque, and utilizes the specific raw material ratio between pullulan as the main film-forming material and an auxiliary film-forming material to enable the film agent to produce uniform microbubbles, thereby rendering the film white and opaque, avoiding the addition of any colorants and opacifiers, and reducing the impact on the active ingredient due to the introduction of new excipients.

A second object of the present disclosure is to provide a microbubble film agent as a substitute for titanium dioxide and a method for preparing the same. The preparation method is simple to operate, has mild operating conditions, and employs different stirring speeds and stirring times during the preparation process to enable the film agent to produce microbubbles with small and uniform particle sizes.

To achieve the above objects of the present disclosure, the following technical solutions are adopted.

The present disclosure provides a microbubble film agent as a substitute for titanium dioxide, which is mainly prepared from the following raw materials in percentage by mass:
0.01%-40% of an active ingredient;
20%-80% of a high molecular film-forming material;
0.1%-20% of a surfactant; and
2.5%-30% of a thickener;
wherein the high molecular film-forming material comprises pullulan as a main film-forming material and an auxiliary film-forming material; and
a mass ratio of pullulan as the main film-forming material to the auxiliary film-forming material is 1:3 to 3:1.

As a further specific embodiment, the auxiliary film-forming material comprises one or more selected from the group consisting of hydroxypropyl methyl cellulose, hydroxypropyl ethyl cellulose, polyvinyl alcohol, hydroxypropyl cellulose, hydroxyethyl cellulose, polyvinylpyrrolidone, polyvinyl alcohol-polyethylene glycol graft copolymer, maltodextrin, polyethylene oxide, sodium alginate, hydroxypropyl starch, and carbomer.

As a further specific embodiment, the auxiliary film-forming material comprises one or more selected from the group consisting of hydroxypropyl methyl cellulose, polyvinyl alcohol, hydroxypropyl cellulose, polyvinylpyrrolidone, polyvinyl alcohol-polyethylene glycol graft copolymer, maltodextrin, sodium alginate, and hydroxypropyl starch.

As a further specific embodiment, the auxiliary film-forming material is hydroxypropyl methyl cellulose, hydroxypropyl cellulose, and polyvinyl alcohol.

As a further specific embodiment, a mass ratio of pullulan to the auxiliary film-forming material is in a range from 2:1 to 1:3.

As a further specific embodiment, a mass ratio of pullulan to the auxiliary film-forming material is 2:3.

As a further specific embodiment, a mass ratio of hydroxypropyl methyl cellulose : hydroxypropyl cellulose : polyvinyl alcohol is 1:1:1.

As a further specific embodiment, the thickener is one or more selected from the group consisting of a low molecular thickener and a macromolecular suspending agent;
wherein the low molecular thickener is one or more selected from the group consisting of sucrose, maltose, fructose, mannitol, glucose, lactose, trehalose, xylitol, maltitol, erythritol, sorbitol, lactitol, cetyl palmitate, and stearic acid;
the macromolecular suspending agent is one or more selected from the group consisting of arabic gum, agar, carrageenan, dextrin, gellan gum, guar gum, pectin, propylene glycol alginate, sodium carboxymethyl cellulose, starch, gelatin, tragacanth, xanthan gum, and gum.

As a further specific embodiment, a mass ratio of the high molecular film-forming material to the thickener is 20:1 to 1:1.

As a further specific embodiment, the low molecular thickener is one or more selected from the group consisting of maltose, mannitol, glucose, lactose, trehalose, xylitol, maltitol, and erythritol; and
the macromolecular suspending agent is one or more selected from the group consisting of carrageenan, dextrin, gellan gum, guar gum, pectin, propylene glycol alginate, sodium carboxymethyl cellulose, xanthan gum, and gum.

As a further specific embodiment, the thickener is a mixture of maltose as the low molecular thickener and dextrin as the macromolecular suspending agent.

As a further specific embodiment, a mass ratio of maltose to dextrin is 2:3.

As a further specific embodiment, the mass ratio of the high molecular film-forming material to the thickener is 10:1 to 1.2:1.

As a further specific embodiment, the surfactant is one or more selected from the group consisting of sodium dodecyl sulfate, Tween, poloxamer, lecithin, and fatty acid glycerides.

As a further specific embodiment, the surfactant is sodium dodecyl sulfate and Tween 80.

As a further specific embodiment, a mass ratio of sodium dodecyl sulfate : Tween 80 is 2:3.

As a further specific embodiment, 0-35% of other excipients are further comprised, the other excipients comprising one or more selected from the group consisting of disintegrants, plasticizers, stabilizers, colorants, and flavoring agents.

As a further specific embodiment, the disintegrant is one or more selected from the group consisting of crospovidone, sodium carboxymethyl cellulose, and corn starch.

As a further specific embodiment, the plasticizer is one or more selected from the group consisting of glycerol, PEG, and triacetin.

As a further specific embodiment, the stabilizer is one or more selected from the group consisting of hydroxypropyl-β-cyclodextrin, hydroxybutyl-β-cyclodextrin, and sulfobutyl-β-cyclodextrin, BHT, EDTA, BHA, sodium bisulfite, and sodium metabisulfite;
the colorant is one or more selected from the group consisting of ferric oxide, tartrazine, amaranth, and brilliant blue;
the flavoring agent is one or more selected from the group consisting of sucralose, aspartame, stevioside, peppermint flavor, strawberry flavor, and sweet orange flavor.

For the raw materials above, the active ingredient is any drug that can be dissolved or dispersed in water, including but not limited to: one or more of the drugs, such as diazepam, dexmedetomidine, riluzole, clobazam, fentanyl, buprenorphine, naloxone, voglibose, rizatriptan benzoate, zolmitriptan, zolpidem tartrate, apomorphine hydrochloride, loratadine, desloratadine, rupatadine fumarate, cariprazine hydrochloride, nitroglycerin, calcitriol, alfacalcidol, paricalcitol, vitamin D, vitamin A, sildenafil citrate, tadalafil, vardenafil, racecadotril, ondansetron hydrochloride, ondansetron, granisetron hydrochloride, donepezil hydrochloride, aripiprazole, asenapine maleate, selegiline hydrochloride, rasagiline mesylate, memantine hydrochloride, olanzapine, montelukast sodium, perampanel, brexpiprazole, vaccines or polypeptides; one or more of health product functional factors, such as probiotics, melatonin, vitamins and vitamin analogs, minerals and trace elements, active sugars, active polypeptides, active proteins, and functional oils; one or more of food ingredients and oral hygiene products such as plant and animal extracts, such as menthol, eucalyptol, methyl salicylate, mogroside, musk, or the like.

In the present disclosure, by stirring pullulan as the main film-forming material and the auxiliary film-forming material to generate bubbles, and then adding a surfactant and a thickener followed by high-speed stirring to obtain a microbubble film agent having microbubbles of 1-100µm, these generated microbubbles can render the finally prepared film white and opaque, which can well substitute of titanium dioxide for coloring, thereby enabling the prepared film agent to possess a white opaque appearance, providing a feasible solution for replacing titanium dioxide in film agent formulations, improving safety, and reducing the impact on the active ingredient due to the introduction of new excipients. Moreover, the microbubble film agent of the present disclosure can be thicker than ordinary film agents, providing some room for increased drug loading. Furthermore, the film prepared from the microbubble film agent of the present disclosure has a faster dissolution effect and provides better hand feeling compared to film agents in the prior art. Therefore, through screening of auxiliary film-forming material, it is found in the present disclosure that only when hydroxypropyl methyl cellulose, hydroxypropyl cellulose, and polyvinyl alcohol are selected as the auxiliary film-forming material, and the mass ratio of pullulan : hydroxypropyl methyl cellulose : hydroxypropyl cellulose : polyvinyl alcohol is 2:1:1:1, pullulan as the main film-forming material and the auxiliary film-forming material can form a large number of bubbles during low-speed stirring, with significantly better foamability than other mixtures of pullulan and other auxiliary film-forming materials. Furthermore, when subsequently maltose and dextrin are selected as the thickener, and sodium dodecyl sulfate and Tween 80 as the surfactant, the microbubbles formed after adding the surfactant and thickener followed by high-speed stirring are the most stable and uniform.

Among the above raw materials, the inventors discovered through creative work that when using pullulan as the main film-forming material and the auxiliary film-forming material in a specific mass ratio, it has good foaming ability, and can generate a large number of bubbles upon low-speed stirring at a specific rate, and then can form microbubbles of 1-100 µm upon subsequent high-speed stirring at a specific rate. Under normal circumstances, pullulan and the auxiliary film-forming material are usually not completely miscible to each other, and thus their mixed aqueous solution forms a two-phase system with a miscibility interface between the two phases. Due to the weak interaction between different phases, the miscibility interface usually has low stability and is easily affected by external disturbances, such as mechanical vibration, shear force, etc., leading to phase interface changes and molecular rearrangement of substances, generating a large number of bubbles. Therefore, only when the mass ratio of pullulan as the main film-forming material to the auxiliary film-forming material is in a range from 3:1 to 1:3, optionally from 2:1 to 1:3, can the prepared film agent produce uniform microbubbles. When the proportion of pullulan is too high, the physical properties of the film are poor; when the proportion of pullulan is too low, it is not easy to form a two-phase miscibility interface, and a large number of bubbles cannot be generated. Since the microbubbles themselves have certain instability after generation, the present disclosure provides the microbubbles with better stability by adding a surfactant and a thickener. Moreover, the mass ratio of the added thickener to the high molecular film-forming material is limited. Only when the mass ratio of the high molecular film-forming material to the thickener is in a range from 20:1 to 1:1, optionally from 10:1 to 1.2:1, are the formed microbubbles the most stable. This is because, by adding the surfactant and thickener and using high-speed stirring in the present disclosure, the surfactant can exhibit lower surface tension at the gas-liquid interface, forming a thin film, thereby further facilitating the generation of richer and finer foam, and the thickener can absorb a large amount of water in the mixture, increasing the viscosity of the mixture, thereby improving the mechanical strength of the foam film, hindering foam shrinkage and liquid penetration, and thus improving foam stability. For this reason, when the proportion of thickener is too high, the mixture becomes too viscous, which is not conducive to subsequent high-speed stirring, and the corresponding film-forming material is too little, which is even more detrimental to film formation. When the proportion of thickener is too low, the mixture has a small viscosity, and the foam stability time is short, thereby affecting the size and uniformity of the microbubbles during the film agent production process.

The present disclosure also provides a method for preparing the above-mentioned microbubble film agent as a substitute for titanium dioxide, comprising the following steps:
adding pullulan and other high molecular film-forming materials sequentially with stirring at a low speed of 100-800 r/min for 2-30 min to obtain a mixture; and
adding an active ingredient, a surfactant, and a thickener sequentially to the mixture with stirring at a high speed of 2,000-12,000 r/min for 2-20 min, and then coating and drying to obtain the microbubble film agent.

As a further specific embodiment, the stirring at a low speed is performed at a speed of 200-600 r/min for 5-20 min to obtain the mixture.

As a further specific embodiment, the stirring at a high speed is performed at a speed of 3,000-8,000 r/min for 3-15 min.

In the preparation method of the above microbubble film agent, the stirring speed and stirring time in the two stages are extremely important for the present disclosure. This is because current researches on substitutes for titanium dioxide are mainly based on white materials such as calcium carbonate, zinc oxide, talc, and starch-based products. However, these substitute materials provide weaker whiteness and shielding capability than titanium dioxide, and higher doses are required to achieve comparable effects to titanium dioxide, which may affect the physicochemical properties of the formulation. In addition, some materials proposed as substitutes for titanium dioxide are required to have a particle size in a range between 1-100 µm, which also presents an inhalation carcinogenic risk of nano-sized particles. Therefore, there is an urgent need for a reliable alternative to replace titanium dioxide in orally disintegrating film agents. The inventors accidentally discovered through creative work that a transparent gel slurry free of titanium dioxide can become an opaque white foam after high-speed stirring. Coating and drying the gel slurry under this condition yields a film agent with an opaque white appearance, demonstrating the potential as a substitute for titanium dioxide.

Therefore, in the present disclosure, the microbubble film agent is prepared by specifically selecting the film-forming materials and adopting stepwise stirring process. After adding pullulan as the main film-forming material and the auxiliary film-forming material, the stirring is performed at a low speed in the range of 100-800 r/min, optionally 200-600 r/min, for 2-30 min, optionally 5-20 min. If the stirring speed is too low or the stirring time is too short, it is not conducive to the full mixing between pullulan and the auxiliary film-forming material, affecting subsequent stirring. If the stirring speed is too high, many bubbles of different sizes will be instantly generated upon stirring, resulting in uneven microbubbles and affecting the effect of film agent.

Compared with the prior art, the present disclosure has the following beneficial effects:
(1) The present disclosure provides a microbubble film agent as a substitute for titanium dioxide. This microbubble film agent utilizes the physical scattering effect of microbubbles on light to render the film white and opaque, and utilizes the specific raw material ratio between pullulan as the main film-forming material and the auxiliary film-forming material to produce uniform microbubbles using the film agent, thereby rendering the film white and opaque, providing a feasible solution for replacing titanium dioxide in film agent formulations, and reducing the safety risk of excipients.
(2) The preparation method of the microbubble film agent of the present disclosure is simple to operate under mild conditions. It employs different stirring speeds and stirring times during the preparation process to enable the film agent to produce microbubbles with small and uniform particle sizes.
(3) The microbubble film agent provided by the present disclosure has a faster dissolution speed compared to conventional film agents.

### DETAILED DESCRIPTION

The technical solutions of the present disclosure will be described clearly and completely below with reference to the embodiments of the present disclosure. Obviously, the described embodiments are only a part of the embodiments of the present disclosure, not all of them. Based on the embodiments in the present disclosure, all other embodiments obtained by ordinary skilled persons in the art without creative work shall fall within the protection scope of the present disclosure.

To illustrate the technical solutions in the present disclosure more clearly, the following description is made in the form of specific examples.

### Example 1

The preparation process of the microbubble film agent was as follows:

### Formulation:

Calcitriol as an active ingredient 0.01 g;
Pullulan as a main film-forming material 40 g;
Hydroxypropyl methyl cellulose as an auxiliary film-forming material 40 g;
Sodium dodecyl sulfate as a surfactant 0.1 g;
Sucrose as a thickener 8 g; and
Other excipients 11.89 g.

40 g of pullulan as a main film-forming material and 40 g of hydroxypropyl methyl cellulose as an auxiliary film-forming material were added into a reaction kettle, the stirrer was turned on, and the speed was adjusted to 100 r/min for low-speed stirring for 30 min to obtain a mixture;
0.01 g of calcitriol as an active ingredient, 0.1 g of sodium dodecyl sulfate as a surfactant, 8 g of sucrose as a thickener, and 11.89 g of other excipients were then added to the reaction kettle containing the mixture, the speed was adjusted to 2,000 r/min for high-speed stirring for 30 min to obtain a gel slurry, which was then coated and dried to obtain the microbubble film agent.

### Example 2

The preparation process of the microbubble film agent was as follows:

### Formulation:

Desloratadine as an active ingredient 40 g;
Pullulan as a main film-forming material 10 g;
Hydroxypropyl methyl cellulose as an auxiliary film-forming material 10 g;
Poloxamer as a surfactant 20 g; and
Sucrose as a thickener 20 g.
10 g of pullulan as a main film-forming material and 10 g of hydroxypropyl methyl cellulose as an auxiliary film-forming material were added into a reaction kettle, the stirrer was turned on, and the speed was adjusted to 800 r/min for low-speed stirring for 2 min to obtain a mixture;
40 g of desloratadine as an active ingredient, 20 g of sodium dodecyl sulfate as a surfactant, and 20 g sucrose as a thickener were then added to the reaction kettle containing the mixture, the speed was adjusted to 12,000 r/min for high-speed stirring for 2 min to obtain a gel slurry, which was then coated and dried to obtain the microbubble film agent.

### Example 3

The preparation process of the microbubble film agent was as follows:

### Formulation:

Brexpiprazole as an active ingredient 1 g;
Pullulan as a main film-forming material 20 g;
Hydroxypropyl methyl cellulose as an auxiliary film-forming material 30 g;
Sodium dodecyl sulfate as a surfactant 15 g;
Sucrose as a thickener 30 g; and
Other excipients 4 g.

20 g of pullulan as a main film-forming material and 30 g of hydroxypropyl methyl cellulose as an auxiliary film-forming material were added into a reaction kettle, the stirrer was turned on, and the speed was adjusted to 600 r/min for low-speed stirring for 20 min to obtain a mixture;
1 g of brexpiprazole as an active ingredient, 15 g of sodium dodecyl sulfate as a surfactant, 30 g sucrose as a thickener, and 4 g of other excipients were then added to the reaction kettle containing the mixture, the speed was adjusted to 8,000 r/min for high-speed stirring for 20 min to obtain a gel slurry, which was then coated and dried to obtain the microbubble film agent.

### Example 4

The preparation process of the microbubble film agent was as follows:

### Formulation:

Vitamin D3 1 g;
Pullulan 43.5 g;
Polyvinyl alcohol 7.25 g;
Hydroxypropyl methyl cellulose 7.25 g;
Sodium dodecyl sulfate 1 g;
Maltose 15 g; and
Sulfobutyl-β-cyclodextrin 25 g.

43.5 g of pullulan as a main film-forming material, and 7.25 g of hydroxypropyl methyl cellulose and 7.25 g of polyvinyl alcohol as auxiliary film-forming materials were added into a reaction kettle, the stirrer was turned on, and the speed was adjusted to 100 r/min for low-speed stirring for 30 min to obtain a mixture;
1 g of vitamin D3 as an active ingredient, 1 g of sodium dodecyl sulfate as a surfactant, 15 g of maltose as a thickener, and 25 g of sulfobutyl-β-cyclodextrin were then added to the reaction kettle containing the mixture, the speed was adjusted to 12,000 r/min for high-speed stirring for 2 min to obtain a gel slurry, which was then coated and dried to obtain the microbubble film agent.

### Example 5

The preparation process of the microbubble film agent was as follows:

### Formulation:

Vitamin D 0.01 g;
Vitamin A 0.03 g;
Pullulan 16 g;
Hydroxypropyl cellulose 16 g;
Hydroxypropyl methyl cellulose 16 g;
Tween 80 0.99 g;
Dextrin 20 g;
Hydroxypropyl-β-cyclodextrin 30 g; and
EDTA 0.97 g.

16 g of pullulan as a main film-forming material, and 16 g of hydroxypropyl methyl cellulose and 16 g of hydroxypropyl cellulose as auxiliary film-forming materials were then added into a reaction kettle, the stirrer was turned on, and the speed was adjusted to 300 r/min for low-speed stirring for 20 min to obtain a mixture;
0.01 g of vitamin D and 0.03 g of vitamin A as active ingredients, 20 g of dextrin as a thickener, 0.99 g of Tween 80 as a surfactant, and other excipients (30 g of hydroxypropyl-β-cyclodextrin and 0.97 g of ethylenediaminetetraacetic acid (EDTA) as stabilizers) were then added to the reaction kettle containing the mixture, the speed was adjusted to 10,000 r/min for high-speed stirring for 3 min to obtain a gel slurry, which was then coated and dried to obtain the microbubble film agent.

### Example 6

The preparation process of the microbubble film agent was as follows:

### Formulation:

Tadalafil 40 g;
Pullulan 25 g;
Sodium alginate 8.3 g;
Hydroxypropyl methyl cellulose 16.7 g;
Tween 80 1 g;
Xanthan gum 2.5 g;
Crospovidone 3.3 g;
Strawberry flavor 2 g;
Sucralose 1 g; and
Brilliant blue 0.2 g.
25 g of pullulan as a main film-forming material,and 16.7 g of hydroxypropyl methyl cellulose and 8.3 g of sodium alginate as auxiliary film-forming materials were added into a reaction kettle, the stirrer was turned on, and the speed was adjusted to 800 r/min for low-speed stirring for 2 min to obtain a mixture;
40 g tadalafil as an active ingredient, 1 g of Tween 80, 2.5 g of xanthan gum, 3.3 g of crospovidone, 2 g of strawberry flavor, 1 g of sucralose, and 0.2 g of brilliant blue were then added to the reaction kettle containing the mixture, the speed was adjusted to 2,000 r/min for high-speed stirring for 20 min to obtain a gel slurry, which was then coated and dried to obtain the microbubble film agent.

### Example 7

The preparation process of the microbubble film agent was as follows:

### Formulation:

Melatonin 10 g;
Pullulan 32.4 g;
Hydroxypropyl cellulose 16.25 g;
Hydroxypropyl methyl cellulose 16.25 g;
Sodium dodecyl sulfate 1 g;
Propylene glycol alginate 10 g;
Crospovidone 10 g;
Strawberry flavor 2 g;
Sucralose 2 g; and
Brilliant blue 0.1 g.

32.4 g of pullulan as a main film-forming material, and 16.25 g of hydroxypropyl cellulose and 16.25 g of hydroxypropyl methyl cellulose as auxiliary film-forming materials were added into a reaction kettle, the stirrer was turned on, and the speed was adjusted to 600 r/min for low-speed stirring for 5 min to obtain a mixture;
10 g of melatonin as an active ingredient, 1 g of sodium dodecyl sulfate, 10 g of propylene glycol alginate, 10 g of crospovidone, 2 g of strawberry flavor, 2 g of sucralose, and 0.1 g of brilliant blue were then added to the reaction kettle containing the mixture, the speed was adjusted to 4,000 r/min for high-speed stirring for 15 min to obtain a gel slurry, which was then coated and dried to obtain the microbubble film agent.

### Example 8

The preparation process of the microbubble film agent was as follows:

### Formulation:

Menthol 5 g;
Eucalyptol 5 g;
Methyl salicylate 5 g;
Pullulan 30 g;
Sodium alginate 10 g;
Maltodextrin 10 g;
Poloxamer 3 g;
Pectin 10 g;
Crospovidone 15 g;
Strawberry flavor 3 g; and
Stevioside 4 g.

30 g of pullulan as a main film-forming material, and 10 g of sodium alginate 10 g and 10 g of maltodextrin as auxiliary film-forming materials were then added into a reaction kettle, the stirrer was turned on, and the speed was adjusted to 600 r/min for low-speed stirring for 5 min to obtain a mixture;
5 g of menthol, 5 g of eucalyptol and 5 g of methyl salicylate as active ingredients, 1 g of poloxamer, 10 g of pectin, 15 g of crospovidone, 3 g of strawberry flavor, and 4 g of stevioside were then added to the reaction kettle containing the mixture, the speed was adjusted to 8,000 r/min for high-speed stirring for 12 min to obtain a gel slurry, which was then coated and dried to obtain the microbubble film agent.

### Example 9

The preparation process of the microbubble film agent was as follows:

### Formulation:

Sodium hyaluronate 10 g;
Pullulan 20 g;
Hydroxypropyl ethyl cellulose 10 g;
Hydroxypropyl cellulose 10 g;
Lecithin 5 g;
Trehalose 30 g;
Glycerol 5 g;
Peppermint flavor 5 g;
Sucralose 4 g; and
Tartrazine 1 g.

20 g of pullulan as a main film-forming material and 10 g of hydroxypropyl ethyl cellulose and 10 g of hydroxypropyl cellulose as auxiliary film-forming materials were added into a reaction kettle, the stirrer was turned on, and the speed was adjusted to 500 r/min for low-speed stirring for 6 min to obtain a mixture;
10 g of sodium hyaluronate as a active ingredient, 5 g of lecithin, 30 g of trehalose, 5 g of glycerol, 5 g of peppermint flavor, 4 g of sucralose, and 1 g of tartrazine were then added to the reaction kettle containing the mixture, the speed was adjusted to 6,000 r/min for high-speed stirring for 10 min to obtain a gel slurry, which was then coated and dried to obtain the microbubble film agent.

### Example 10

The preparation process of the microbubble film agent was as follows:

### Formulation:

Rupatadine fumarate 10 g;
Pullulan 17.4 g;
Hydroxypropyl methyl cellulose 17.5 g;
Sodium dodecyl sulfate 0.1 g;
Maltitol 25 g; and
HP-β-cyclodextrin 30 g.

17.4 g of pullulan as a main film-forming material and 17.4 g of hydroxypropyl methyl cellulose as an auxiliary film-forming material were added into a reaction kettle, the stirrer was turned on, and the speed was adjusted to 600 r/min for low-speed stirring for 5 min to obtain a mixture;
10 g of rupatadine fumarate as an active ingredient, 0.1 g of sodium dodecyl sulfate, 25 g of maltitol, and 30 g of HP-β-cyclodextrin were then added to the reaction kettle containing the mixture, the speed was adjusted to 5,000 r/min for high-speed stirring for 10 min to obtain a gel slurry, which was then coated and dried to obtain the microbubble film agent.

### Example 11

The preparation process of the microbubble film agent was as follows:

### Formulation:

Cariprazine hydrochloride 15 g;
Pullulan 25.8 g;
Polyvinyl alcohol 25.3 g;
Polyvinylpyrrolidone 12.7 g;
Poloxamer 0.1 g;
Arabic gum 10 g;
Triacetin 10 g;
Butylated hydroxytoluene (BHT) 0.1 g; and
Aspartame 1 g.

25.8 g of pullulan as a main film-forming material and 25.3 g of polyvinyl alcohol and 12.7 g of polyvinylpyrrolidone as auxiliary film-forming materials were then added into a reaction kettle, the stirrer was turned on, and the speed was adjusted to 500 r/min for low-speed stirring for 6 min to obtain a mixture;
15 g of cariprazine hydrochloride as an active ingredient, 0.1 g of poloxamer, 10 g of arabic gum, 10 g of triacetin, 0.1 g of BHT, and 1 g of aspartame were then added to the reaction kettle containing the mixture, the speed was adjusted to 8,000 r/min for high-speed stirring for 12 min to obtain a gel slurry, which was then coated and dried to obtain the microbubble film agent.

### Example 12

The preparation process of the microbubble film agent was as follows:

### Formulation:

Nitroglycerin 3 g;
Pullulan 33 g;
Polyvinyl alcohol 24.75 g;
Hydroxypropyl starch 8.25 g;
Fatty acid glyceride 5 g;
Sucrose 20 g;
Sodium bisulfite 2 g;
Sucralose 2 g; and
Peppermint flavor 2 g.

33 g of pullulan as a main film-forming material, and 24.75 g of polyvinyl alcohol 24.75 g and 8.25 g of hydroxypropyl starch as auxiliary film-forming materials were added into a reaction kettle, the stirrer was turned on, and the speed was adjusted to 500 r/min for low-speed stirring for 6 min to obtain a mixture;
3 g of nitroglycerin as an active ingredient, 20 g of sucrose, 2 g of sodium bisulfite, 2 g of sucralose, and 2 g of peppermint flavor were then added to the reaction kettle containing the mixture, the speed was adjusted to 5,000 r/min for high-speed stirring for 15 min to obtain a gel slurry, which was then coated and dried to obtain the microbubble film agent.

### Example 13

The preparation process of the microbubble film agent was as follows:

### Formulation:

Selegiline hydrochloride 5 g;
Pullulan 40 g;
Carbomer 4.43 g;
Hydroxypropyl methyl cellulose 35.47 g;
Poloxamer 5 g;
Gellan gum 5 g;
Stevioside 5 g; and
Brilliant blue 0.1 g.

40 g of pullulan as a main film-forming material, and 4.43 g of carbomer and 35.47 g of hydroxypropyl methyl cellulose as auxiliary film-forming materials were added into a reaction kettle, the stirrer was turned on, and the speed was adjusted to 700 r/min for low-speed stirring for 5 min to obtain a mixture;
5 g of selegiline hydrochloride as an active ingredient, 5 g of poloxamer, 5 g of gellan gum, 5 g of stevioside, and 0.1 g of brilliant blue were then added to the reaction kettle containing the mixture, the speed was adjusted to 6,000 r/min for high-speed stirring for 18 min to obtain a gel slurry, which was then coated and dried to obtain the microbubble film agent.

### Example 14

The specific preparation steps of the microbubble film agent were the same as those in Example 3, except that the auxiliary film-forming material was replaced with hydroxypropyl ethyl cellulose, with the mass of the auxiliary film-forming material itself unchanged.

### Example 15

The specific preparation steps of the microbubble film agent were the same as those in Example 3, except that the auxiliary film-forming material was replaced with hydroxyethyl cellulose, with the mass of the auxiliary film-forming material itself unchanged.

### Example 16

The specific preparation steps of the microbubble film agent were the same as those in Example 3, except that the auxiliary film-forming material was replaced with polyvinyl alcohol, with the mass of the auxiliary film-forming material itself unchanged.

### Example 17

The specific preparation steps of the microbubble film agent were the same as those in Example 3, except that the auxiliary film-forming material was replaced with polyvinyl alcohol-polyethylene glycol graft copolymer, with the mass of the auxiliary film-forming material itself unchanged.

### Example 18

The specific preparation steps of the microbubble film agent were the same as those in Example 3, except that the auxiliary film-forming material was replaced with hydroxypropyl ethyl cellulose and carbomer, with the mass of the auxiliary film-forming material itself unchanged, wherein 15 g of hydroxypropyl ethyl cellulose and 15 g of carbomer were added.

### Example 19

The specific preparation steps of the microbubble film agent were the same as those in Example 3, except that the auxiliary film-forming material was replaced with hydroxypropyl ethyl cellulose, carbomer, and hydroxypropyl starch, with the mass of the auxiliary film-forming material itself unchanged, wherein 10 g of hydroxypropyl ethyl cellulose, 10 g of carbomer, and 10 g of hydroxypropyl starch were added.

### Example 20

The specific preparation steps of the microbubble film agent were the same as those in Example 3, except that the auxiliary film-forming material was replaced with polyvinyl alcohol and sodium alginate, with the mass of the auxiliary film-forming material itself unchanged, wherein 15 g of polyvinyl alcohol and 15 g of sodium alginate were added.

### Example 21

The specific preparation steps of the microbubble film agent were the same as those in Example 3, except that the auxiliary film-forming material was replaced with polyvinyl alcohol, sodium alginate, and hydroxypropyl starch, with the mass of the auxiliary film-forming material itself unchanged, wherein 10 g of polyvinyl alcohol, 10 g of sodium alginate, and 10 g of hydroxypropyl starch were added.

### Example 22

The specific preparation steps of the microbubble film agent were the same as those in Example 3, except that the auxiliary film-forming material was replaced with hydroxypropyl methyl cellulose, hydroxypropyl cellulose, and polyvinyl alcohol, and the mass ratio of hydroxypropyl methyl cellulose : hydroxypropyl cellulose : polyvinyl alcohol was 1:1:1, with the mass of the auxiliary film-forming material itself unchanged, wherein 10 g of hydroxypropyl methyl cellulose, 10 g of hydroxypropyl cellulose, and 10 g of polyvinyl alcohol were added.

### Example 23

The specific preparation steps of the microbubble film agent were the same as those in Example 22, except that the mass ratio of hydroxypropyl methyl cellulose : hydroxypropyl cellulose : polyvinyl alcohol was adjusted to 2 : 1 : 1, with the mass of the auxiliary film-forming material itself unchanged, wherein 15 g of hydroxypropyl methyl cellulose, 7.5 g of hydroxypropyl cellulose, and 7.5 g of polyvinyl alcohol were added.

### Example 24

The specific preparation steps of the microbubble film agent were the same as those in Example 22, except that the mass ratio of hydroxypropyl methyl cellulose : hydroxypropyl cellulose : polyvinyl alcohol was adjusted to 3 : 1 : 1, with the mass of the auxiliary film-forming material itself unchanged, wherein 18 g of hydroxypropyl methyl cellulose, 6 g of hydroxypropyl cellulose, and 6 g of polyvinyl alcohol were added.

### Example 25

The specific preparation steps of the microbubble film agent were the same as those in Example 22, except that the thickener was replaced withmaltose, with the mass of the thickener itself unchanged.

### Example 26

The specific preparation steps of the microbubble film agent were the same as those in Example 22, except that the thickener was replaced with sorbitol, with the mass of the thickener itself unchanged.

### Example 27

The specific preparation steps of the microbubble film agent were the same as those in Example 22, except that the thickener was replaced with carrageenan, with the mass of the thickener itself unchanged.

### Example 28

The specific preparation steps of the microbubble film agent were the same as those in Example 22, except that the thickener was replaced with maltose, mannitol, glucose, dextrin, and arabic gum, with the mass of the thickener itself unchanged, wherein 6 g of maltose, 6 g of mannitol, 6 g of glucose, 6 g of dextrin, and 6 g of arabic gum were added.

### Example 29

The specific preparation steps of the microbubble film agent were the same as those in Example 22, except that the thickener was replaced with maltose and dextrin, and the mass ratio of maltose to dextrin was 2 : 3, with the mass of the thickener itself unchanged, wherein 12 g of maltose and 18 g of dextrin were added.

### Example 30

The specific preparation steps of the microbubble film agent were the same as those in Example 29, except that the mass ratio of maltose to dextrin was adjusted to 1 : 3, with the mass of the thickener itself unchanged, wherein 7.5 g of maltose and 22.5 g of dextrin were added.

### Example 31

The specific preparation steps of the microbubble film agent were the same as those in Example 29, except that the surfactant was replaced with Tween, with the mass of the surfactant itself unchanged.

### Example 32

The specific preparation steps of the microbubble film agent were the same as those in Example 29, except that the surfactant was replaced with poloxamer and lecithin, with the mass of the surfactant itself unchanged, wherein 7.5 g of poloxamer and 7.5 g of lecithin were added.

### Example 33

The specific preparation steps of the microbubble film agent were the same as those in Example 29, except that the surfactant was replaced with sodium dodecyl sulfate and Tween 80, with the mass of the surfactant itself unchanged, wherein 6 g of sodium dodecyl sulfate and 9 g of Tween 80 were added.

### Example 34

The specific preparation steps of the microbubble film agent were the same as those in Example 33, except that the surfactant was replaced with sodium dodecyl sulfate and Tween 80, with the mass of the surfactant itself unchanged, wherein 10 g of sodium dodecyl sulfate and 5 g of Tween 80 were added.

### Comparative Example 1

The specific preparation steps of the microbubble film agent were the same as those in Example 33, except that no pullulan as the main film-forming material was used.

### Comparative Example 2

The specific preparation steps of the microbubble film agent were the same as those in Example 33, except that no auxiliary film-forming materials were used.

### Comparative Example 3

The specific preparation steps of the microbubble film agent were the same as those in Example 33, except that no surfactant was used.

### Comparative Example 4

The specific preparation steps of the microbubble film agent were the same as those in Example 33, except that no thickener was used.

### Comparative Example 5

The specific preparation steps of the microbubble film agent were the same as those in Example 33, except that after adding pullulan as the main film-forming material and the auxiliary film-forming materials, the stirring speed was adjusted to 10,000 r/min.

### Comparative Example 6

The specific preparation steps of the microbubble film agent were the same as those in Example 33, except that after adding pullulan as the main film-forming material and the auxiliary film-forming materials, the stirring speed was adjusted to 50 r/min.

### Comparative Example 7

The specific preparation steps of the microbubble film agent were the same as those in Example 33, except that after adding the active ingredient, surfactant, and thickener, the stirring speed was adjusted to 600 r/min.

### Comparative Example 8

The specific preparation steps of the microbubble film agent were the same as those in Example 33, except that after adding the active ingredient, surfactant, and thickener, the stirring speed was adjusted to 15,000 r/min.

### Comparative Example 9

Compared with Example 33, the gel slurry was subjected to vacuum stirring for defoaming treatment before coating and drying, without subsequent two-step stirring, to obtain a conventional film agent without microbubbles.

### Experimental Example 1

The films obtained by coating, drying, and slicing the microbubble film agents from Examples 1-24 and Comparative Examples 1-9 were tested. The test data are shown in Table 1 below, wherein the dissolution time was detected using the disintegration limit test method (General Rule 0921) of the Chinese Pharmacopoeia.

**Table 1 Appearance and Performance of Films**

| Test Item | Appearance | Bubble Diameter (µm) | Dissolution Time (s) |
|---|---|---|---|
| Example 1 | Opaque white film, uniform, smooth | 62 | 34 |
| Example 2 | Opaque white film, uniform, smooth | 70 | 31 |
| Example 3 | Opaque white film, uniform, smooth | 62 | 32 |
| Example 4 | Opaque white film, uniform, smooth | 65 | 33 |
| Example 5 | Opaque white film, uniform, smooth | 75 | 35 |
| Example 6 | Opaque light blue film, uniform, smooth | 64 | 34 |
| Example 7 | Opaque light blue film, uniform, smooth | 68 | 33 |
| Example 8 | Opaque white film, uniform, smooth | 59 | 38 |
| Example 9 | Opaque light yellow film, uniform, smooth | 57 | 35 |
| Example 10 | Opaque white film, uniform, smooth | 68 | 34 |
| Example 11 | Opaque white film, uniform, smooth | 67 | 33 |
| Example 12 | Opaque white film, uniform, smooth | 59 | 35 |
| Example 13 | Opaque light blue film, uniform, smooth | 64 | 31 |
| Example 14 | Opaque white film, uniform, smooth | 68 | 36 |
| Example 15 | Opaque white film, uniform, smooth | 65 | 34 |
| Example 16 | Opaque white film, uniform, smooth | 67 | 33 |
| Example 17 | Opaque white film, uniform, smooth | 69 | 34 |
| Example 18 | Opaque white film, uniform, smooth | 65 | 32 |
| Example 19 | Opaque white film, uniform, smooth | 70 | 35 |
| Example 20 | Opaque white film, uniform, smooth | 68 | 32 |
| Example 21 | Opaque white film, uniform, smooth | 65 | 33 |
| Example 22 | Opaque white film, uniform, smooth | 55 | 25 |
| Example 23 | Opaque white film, uniform, smooth | 66 | 32 |
| Example 24 | Opaque white film, uniform, smooth | 65 | 32 |
| Example 25 | Opaque white film, uniform, smooth | 62 | 31 |
| Example 26 | Opaque white film, uniform, smooth | 61 | 33 |
| Example 27 | Opaque white film, uniform, smooth | 68 | 34 |
| Example 28 | Opaque white film, uniform, smooth | 69 | 35 |
| Example 29 | Opaque white film, uniform, smooth | 55 | 26 |
| Example 30 | Opaque white film, uniform, smooth | 66 | 36 |
| Example 31 | Opaque white film, uniform, smooth | 58 | 28 |
| Example 32 | Opaque white film, uniform, smooth | 52 | 25 |
| Example 33 | Opaque white film, uniform, smooth | 45 | 18 |
| Example 34 | Opaque white film, uniform, smooth | 51 | 24 |
| Comparative Example 1 | Uneven large bubbles visible on film surface, locally transparent | 123 | 72 |
| Comparative Example 2 | brittle Film, visible surface cracks, visible uneven large bubbles, locally transparent | 129 | 71 |
| Comparative Example 3 | Uneven large bubbles visible on film surface, locally transparent | 145 | 70 |
| Comparative Example 4 | Uneven large bubbles visible on film surface, locally transparent | 127 | 79 |
| Comparative Example 5 | Uneven large bubbles visible on film surface, locally transparent | 135 | 74 |
| Comparative Example 6 | Uneven large bubbles visible on film surface, locally transparent | 115 | 73 |
| Comparative Example 7 | Uneven large bubbles visible on film surface, locally transparent | 144 | 76 |
| Comparative Example 8 | Uneven large bubbles visible on film surface, locally transparent | 125 | 77 |
| Comparative Example 9 | Colorless transparent film, difficult to identify | - | 72 |

Based on the above data, the following conclusions can be drawn:
From the data in the table, it could be known that the effects of the microbubble film agents prepared according to the solutions of Examples 1-34 were all superior to those of the microbubble film agents prepared in Comparative Examples 1-9. The microbubble film agent prepared according to the solution of Example 33 had the most excellent performance and possessed good foaming ability due to the specific mass ratio between pullulan and the auxiliary high molecular materials. After low-speed stirring, a large number of bubbles could be generated, andmicrobubbles with a diameter of 1-100 µm could be then formed after adding the surfactant and thickner and undergoing high-speed stirring, thereby enabling the prepared film to present an opaque white color without adding titanium dioxide, and could replace titanium dioxide in orally disintegrating film agents. The present disclosure utilizes the physical phenomenon of light scattering by microbubbles, thereby enabling the film agent to possess a white opaque appearance, providing a feasible solution for replacing titanium dioxide in film agent formulations, avoiding the addition of any colorants and opacifiers, reducing the impact on the active ingredient due to the introduction of new excipients. Moreover, the film agent according to the present application can be thicker than ordinary films, thus providing some room for increased drug loading. Furthermore, the microbubble film agent of the present disclosure has a faster dissolution speed compared to the film agent prepared by drying and coating the defoamed control group, but can also be thicker than ordinary film agents, with a good hand feel, further improving identification.

By comparing the microbubble film agents of Examples 14-24 and comparative examples 1-2, it could be known that the main film-forming material and auxiliary film-forming materials used in the present disclosure had a certain impact on the final effect of the prepared microbubble film agent. From the experimental data of Example 22, it could be known that the appearance of the microbubble film agent prepared in Example 22 could present an opaque white color, compared to Examples 14-21. Moreover, by mixing pullulan as the main film-forming material and hydroxypropyl methyl cellulose, hydroxypropyl cellulose, and polyvinyl alcohol as the auxiliary film-forming materials, the microbubble film agent achieved quite excellent foaming ability, thereby enabling the generation of microbubbles with smaller bubble diameters in subsequent stirring. Therefore, by using the mixture of pullulan as the main film-forming material and hydroxypropyl methyl cellulose, hydroxypropyl cellulose, and polyvinyl alcohol as the auxiliary film-forming materials, the microbubble film agent prepared in Example 22 had a formed bubble diameter of 55µm and was significantly better than that of Examples 14-21. Due to the smaller bubble diameter, the dissolution time of the formed film was also improved to a certain extent, so Example 22 also performed better in terms of film dissolution time compared to Examples 14-21. By comparing Example 22 and Examples 23-24, it could be known that when pullulan was selected as the main film-forming material and hydroxypropyl methyl cellulose, hydroxypropyl cellulose, and polyvinyl alcohol were selected as the auxiliary film-forming materials, the mass ratio between the auxiliary film-forming materials also affected the effect of the microbubble film agent. From the experimental data, it could be known that when the mass ratio between the auxiliary film-forming materials was 1:1:1, the prepared microbubble film agent had excellent foaming ability, thus a large number of bubbles could be generated during stirring, and uniform and dense microbubbles were formed through secondary high-speed stirring, enabling the prepared film to present an opaque white color. This is because, it is found in the present disclosure through long-term creative work that only by selecting suitable auxiliary film-forming materials, and when pullulan and the auxiliary film-forming materials are at a suitable mass ratio, their uniform mixture could generate good foaming ability after low-speed stirring, and further result in uniform and numerous microbubbles during subsequent high-speed stirring. Therefore, from the experimental data of Examples 14-24, it could be known that when hydroxypropyl methyl cellulose, hydroxypropyl cellulose, and polyvinyl alcohol were selected as the auxiliary film-forming materials, and the mass ratio of pullulan : hydroxypropyl methyl cellulose : hydroxypropyl cellulose : polyvinyl alcohol was 2:1:1:1, the prepared film agent could produce uniform microbubbles. This is because when the proportion of pullulan is too high, the film has poor physical properties, and when the proportion of pullulan is too low, it is difficult to form a two-phase miscibility interface, and no large number of bubbles could be generated.

By comparing Examples 24-30 and Comparative Example 4, it could be known that the selection of the thickener was extremely important for the present disclosure. From the experimental data of Examples 24-30, it could be known that when maltose and dextrin were selected as the thickener, the prepared microbubble film agent was significantly better than other selections in terms of bubble diameter and film dissolution time. The bubble diameter in Example 29 was 55 µm, and the film dissolution time was 26 s, which were better than those in Examples 24-28. Therefore, it could be known that only when the mixture of maltose and dextrin was selected as the thickener, the prepared microbubble film agent could have the optimal effect. By comparing Examples 29-30, it could be known that the mass ratio between maltose and dextrin was also very important for the present disclosure. From the experimental data, it could be known that when the mass ratio between maltose and dextrin in Example 30 was 1:3, the prepared microbubble film agent was inferior in bubble diameter to Example 29 where the mass ratio between maltose and dextrin was 2:3. This is because the microbubbles themselves, being unstable after formation, are provided withbetter stability through the addition of a thickener according to the present disclosure . Moreover, the mass ratio of the added thickener to the total mass of pullulan as the main film-forming material and the auxiliary film-forming materials was limited. Only when the mass ratio of the total mass of pullulan as the main film-forming material and the auxiliary film-forming materials to the thickener was in a range from 20:1 to 1:1, optionally from 10 : 1 to 1.2 : 1, the formed microbubbles were the most stable. This is because the thickener could absorb a large amount of water in the mixture, increasing the viscosity of the mixture, thereby improving the mechanical strength of the foam film, hindering foam shrinkage and liquid penetration, and thus improving foam stability. For this reason, when the proportion of thickener is too high, the mixture become too viscous, which is not conducive to subsequent high-speed stirring, and the corresponding film-forming material was too little, which was even more detrimental to film formation. When the proportion of thickener is too low, the mixture has a small viscosity, and the foam stability time is short, thereby affecting the size and uniformity of the microbubbles during production of the film agent.

By comparing Examples 31-34 and Comparative Example 3, it could be known that the selection of the surfactant was also very important for the present disclosure. From Example 33, it could be known that when sodium dodecyl sulfate and Tween 80 were selected as the surfactant, the prepared microbubble film agent was significantly better than other examples and comparative examples in terms of bubble diameter. This is because, in the present disclosure, adding the surfactant and high-speed stirring allow the gas-liquid interface to exhibit lower surface tension, forming a thin film, thereby further facilitating the generation of richer and finer foam.

By comparing Example 33 and Comparative Examples 5-8, it could be known that the stirring speed after adding pullulan as the main film-forming material and the auxiliary film-forming materials had a certain impact. From the experimental data of Example 33, it could be known that when the stirring speed was within a suitable range, the formed microbubble diameter was significantly better than Comparative Examples 6-9. Therefore, only when the mixture of pullulan and the auxiliary film-forming materials was stirred at a low speed in the range from 100 to 800 r/min, a large number of uniform bubbles could be generated, and then after high-speed stirring at 2,000-12,000 r/min, microbubbles could be formed. Therefore, the stirring speed was very important for the present disclosure. This is because when the stirring speed is too low, it is not conducive to the full mixing between pullulan and the auxiliary film-forming materials, affecting subsequent stirring. If the stirring speed is too high, many bubbles of different sizes would be instantly generated upon stirring, resulting in uneven microbubbles and affecting the effect of the film agent.

By comparing Example 33 and Comparative Examples 1-9, it could be known that the microbubble diameter of the present disclosure had an impact on the appearance of the film. The size uniformity and diameter below 100µm of the microbubbles were very important for the present disclosure. This is because the opaque white color is due to the scattering effect of microbubbles on light. The smaller the bubble diameter, the more light is scattered, and the film looks opaque; the larger the bubble diameter, the more light is transmitted, and the film looks transparent.

The technical features of the above-described embodiments can be combined arbitrarily. For the sake of brevity, not all possible combinations of the technical features in the above embodiments are described. However, as long as there is no contradiction in the combination of these technical features, they should be considered as within the scope of this description.

The above-described embodiments only represent several implementation modes of the present disclosure, and the description thereof is specific and detailed, but should not be construed as limiting the scope of the patent. It should be pointed out that for those of ordinary skill in the art, several modifications and improvements can be made without departing from the concept of the present disclosure, and these all fall within the protection scope of the present disclosure. Therefore, the protection scope of the patent of the present disclosure shall be subject to the appended claims.

## Claims

1. A microbubble film agent as a substitute for titanium dioxide, which is mainly prepared from the following raw materials in percentage by mass:
0.01%-40% of an active ingredient;
20%-80% of a high molecular film-forming material;
0.1%-20% of a surfactant; and
2.5%-30% of a thickener;
wherein the high molecular film-forming material comprises pullulan as a main film-forming material and an auxiliary film-forming material; and a mass ratio of pullulan as the main film-forming material to the auxiliary film-forming material is in a range from 1:3 to 3:1.

2. The microbubble film agent according to claim 1, wherein the auxiliary film-forming material comprises one or more selected from the group consisting of hydroxypropyl methyl cellulose, hydroxypropyl ethyl cellulose, polyvinyl alcohol, hydroxypropyl cellulose, hydroxyethyl cellulose, polyvinylpyrrolidone, polyvinyl alcohol-polyethylene glycol graft copolymer, maltodextrin, polyethylene oxide, sodium alginate, hydroxypropyl starch, and carbomer.

3. The microbubble film agent according to claim 2, wherein the auxiliary film-forming materials comprise one or more selected from the group consisting of hydroxypropyl methyl cellulose, polyvinyl alcohol, hydroxypropyl cellulose, polyvinylpyrrolidone, polyvinyl alcohol-polyethylene glycol graft copolymer, maltodextrin, sodium alginate, and hydroxypropyl starch.

4. The microbubble film agent according to any one of claims 2-3, wherein the auxiliary film-forming material is hydroxypropyl methyl cellulose, hydroxypropyl cellulose, and polyvinyl alcohol.

5. The microbubble film agent according to claim 4, wherein a mass ratio of pullulan to the auxiliary film-forming material is in a range from 2:1 to 1:3.

6. The microbubble film agent according to any one of claims 4-5, wherein a mass ratio of pullulan to the auxiliary film-forming material is 2:3.

7. The microbubble film agent according to claim 4, wherein a mass ratio of hydroxypropyl methyl cellulose : hydroxypropyl cellulose : polyvinyl alcohol is 1:1:1.

8. The microbubble film agent according to any one of claims 1-7, wherein the thickener is one or more selected from the group consisting of a low molecular thickener and a macromolecular suspending agent;
wherein the low molecular thickener is one or more selected from the group consisting of sucrose, maltose, fructose, mannitol, glucose, lactose, trehalose, xylitol, maltitol, erythritol, sorbitol, lactitol, cetyl palmitate, and stearic acid; and
the macromolecular suspending agent is one or more selected from the group consisting of arabic gum, agar, carrageenan, dextrin, gellan gum, guar gum, pectin, propylene glycol alginate, sodium carboxymethyl cellulose, starch, gelatin, tragacanth, xanthan gum, and gum.

9. The microbubble film agent according to claim 8, wherein a mass ratio of the high molecular film-forming material to the thickener is in a range from 20:1 to 1:1.

10. The microbubble film agent according to any one of claims 8-9, wherein the low molecular thickener is one or more selected from the group consisting of maltose, mannitol, glucose, lactose, trehalose, xylitol, maltitol, and erythritol; and
the macromolecular suspending agent is one or more selected from the group consisting of carrageenan, dextrin, gellan gum, guar gum, pectin, propylene glycol alginate, sodium carboxymethyl cellulose, xanthan gum, and gum.

11. The microbubble film agent according to claim 10, wherein the thickener is a mixture of maltose as the low molecular thickener and dextrin as the macromolecular suspending agent.

12. The microbubble film agent according to any one of claims 10-11, wherein a mass ratio of the maltose to the dextrin is 2:3.

13. The microbubble film agent according to any one of claims 10-12, wherein the mass ratio of the high molecular film-forming material to the thickener is 10:1 to 1.2:1.

14. The microbubble film agent according to any one of claims 1-13, wherein the surfactant is one or more selected from the group consisting of sodium dodecyl sulfate, Tween, poloxamer, lecithin, and fatty acid glycerides.

15. The microbubble film agent according to claim 14, wherein the surfactant is sodium dodecyl sulfate and Tween 80.

16. The microbubble film agent according to any one of claims 14-15, wherein a mass ratio of the sodium dodecyl sulfate : Tween 80 is 2:3.

17. The microbubble film agent according to any one of claims 1-16, further comprising 0-35% of other excipients, the other excipients comprising one or more selected from the group consisting of a disintegrant, a plasticizer, a stabilizer, a colorant, and a flavoring agent.

18. The microbubble film agent according to claim 17, wherein the disintegrant is one or more selected from the group consisting of crospovidone, sodium carboxymethyl cellulose, and corn starch.

19. The microbubble film agent according to any one of claims 17-18, wherein the plasticizer is one or more selected from the group consisting of glycerol, PEG, and triacetin.

20. The microbubble film agent according to any one of claims 17-19, wherein the stabilizer is one or more selected from the group consisting of hydroxypropyl-β-cyclodextrin, hydroxybutyl-β-cyclodextrin, and sulfobutyl-β-cyclodextrin, BHT, EDTA, BHA, sodium bisulfite, and sodium metabisulfite;
the colorant is one or more selected from the group consisting of ferric oxide, tartrazine, amaranth, and brilliant blue; or
the flavoring agent is one or more selected from the group consisting of sucralose, aspartame, stevioside, peppermint flavor, strawberry flavor, and sweet orange flavor.

21. A method for preparing a microbubble film agent according to any one of claims 1-20, comprising the following steps:
adding pullulan and other high molecular film-forming materials sequentially with stirring at a low speed of 100-800 r/min for 2-30 min to obtain a mixture; and
adding an active ingredient, a surfactant, and a thickener sequentially to the mixture with stirring at a high speed of 2,000-12,000 r/min for 2-20 min, and then coating and drying to obtain the microbubble film agent.

22. The method according to claim 21, wherein the stirring at a low speed is performed at a speed of 200-600 r/min for 5-20 min to obtain the mixture.

23. The method according to any one of claims 21-22, wherein the stirring at a high speed is performed at a speed of 3000-8000 r/min for 3-15 min.
